(19) Europäisches Patentamt
European Patent Office
Office européen des brevets

(11) Veröffentlichungsnummer: **0 047 405**
**B1**

(12) # EUROPÄISCHE PATENTSCHRIFT

(45) Veröffentlichungstag der Patentschrift: **20.06.84**

(21) Anmeldenummer: **81106376.7**

(22) Anmeldetag: **17.08.81**

(51) Int. Cl.³: **C 07 D 249/08**, A 01 N 43/64, C 07 D 233/60, C 07 D 233/61, A 01 N 43/50

(54) **Azolylalkyl-Derivate, Verfahren zu ihrer Herstellung und ihre Verwendung als Fungizide.**

(30) Priorität: **27.08.80 DE 3032326**

(43) Veröffentlichungstag der Anmeldung: **17.03.82 Patentblatt 82/11**

(45) Bekanntmachung des Hinweises auf die Patenterteilung: **20.06.84 Patentblatt 84/25**

(84) Benannte Vertragsstaaten: **AT BE CH DE FR GB IT LI NL**

(56) Entgegenhaltungen:
**EP - A - 0 007 505**
**EP - A - 0 007 506**
**DE - A - 2 734 426**
**FR - A - 2 300 081**
**US - A - 3 872 117**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Krämer, Wolfgang, Dr., Am Eckbusch 39/45, D-5600 Wuppertal 1 (DE)**
Erfinder: **Büchel, Karl-Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen 1 (DE)**

EP 0 047 405 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Azolylalkylderivate, mehrere Verfahren zu ihrer Herstellung und ihre Verwendung als Fungizide.

Es ist bereits bekannt geworden, dass verschiedene Azolderivate, wie beispielsweise 1-(4-Chlorphenyl)-4,4-dimethyl-2-(1,2,4-triazol-1-yl)pentan-3-on, 2-Acetoxy-3,3-dimethyl-1-(3-trifluormethylphenoxy)-1-(1,2,4-triazol-1-yl)butan, 1-Triphenylmethylimidazol oder 1-(4-Chlorphenoxy)-2-(4-chlorbenzyloxy)-3,3-dimethyl-1-imidazol-1-ylbutan gute fungizide Eigenschaften aufweisen (vgl. DE-OS Nrn. 2734426, 2600799, 2720949 und US-PS Nr. 3321366). Die Wirkung dieser Verbindungen ist jedoch, insbesondere bei niedrigen Aufwandmengen und -konzentrationen, nicht immer ganz befriedigend. Weiterhin sind im Patentschrifttum fungizid wirksame substituierte Alkylimidazole (vgl. FR-A Nr. 2300081) und α-Azolyl-β-hydroxyketone und deren Vorprodukte (vgl. EP-A Nrn. 0007505 und 0007506) offenbart. Die Wirkung der genannten Stoffklassen gegen phytopathogene Pilze ist jedoch nicht immer voll zufriedenstellend.

Es wurden neue Azolylalkylderivate der allgemeinen Formel:

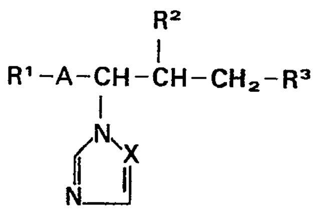

(I)

in welcher

$R^1$ für Alkyl mit 1 bis 4 Kohlenstoffatomen oder für Phenyl steht, wobei letzteres durch Halogen, Alkyl mit 1 bis 4 Kohlenstoffatomen, Halogenalkyl mit 1 oder 2 Kohlenstoff- und 1 bis 5 Halogenatomen, durch Alkoxy und Alkylthio mit jeweils 1 oder 2 Kohlenstoffatomen sowie durch Phenyl substituiert sein kann,

$R^2$ für Wasserstoff, für Alkyl mit 1 bis 4 Kohlenstoffatomen oder für Phenyl steht, wobei letzteres durch die für $R^1$ genannten Phenylsubstituenten substituiert sein kann,

$R^3$ für Cyano oder die Gruppierung $-CO-OR^4$ steht, wobei $R^4$ für Alkyl mit 1 bis 4 Kohlenstoffatomen oder für Benzyl steht, wobei letzteres durch die für $R^1$ genannten Phenylsubstituenten substituiert sein kann,

A für die Ketogruppe oder die CH(OH)-Gruppierung steht, und

X für ein Stickstoffatom oder die CH-Gruppe steht,

sowie deren physiologisch verträgliche Säureadditionssalze und Metallsalzkomplexe gefunden.

Die Verbindungen der Formel I besitzen gegebenenfalls asymmetrische Kohlenstoffatome; sie können dann in verschiedenen geometrischen Isomeren vorliegen, die in unterschiedlichen Mengenverhältnissen anfallen können. In beiden Fällen liegen sie als optische Isomeren vor. Sämtliche Isomeren sind Gegenstand der Erfindung.

Weiterhin wurde gefunden, dass man die Azolylalkylderivate der Formel I erhält, wenn man Azolylketone der Formel:

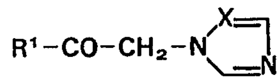

(II)

in welcher $R^1$ und X die oben angegebene Bedeutung haben,

mit Alkenen der Formel:

$$R^2-CH=CH-R^3 \qquad (III)$$

in welcher $R^2$ und $R^3$ die oben angegebene Bedeutung haben,

in Gegenwart eines Verdünnungsmittels und in Gegenwart einer Base sowie gegebenenfalls in Gegenwart eines Katalysators umsetzt und gegebenenfalls die entstehenden Azoylyalkylketoderivate der Formel:

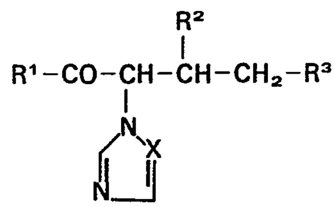

(Ia)

in welcher $R^1$, $R^2$, $R^3$ und X die oben angegebene Bedeutung haben,

nach bekannten Methoden in üblicher Weise reduziert.

An die so erhaltenen Verbindungen der Formel I können gegebenenfalls anschliessend eine Säure oder ein Metallsalz addiert werden. In manchen Fällen erweist es sich als vorteilhaft, die Verbindungen der Formel I über ihre Salze in reiner Form zu erhalten.

Die neuen Azolylalkylderivate der Formel:

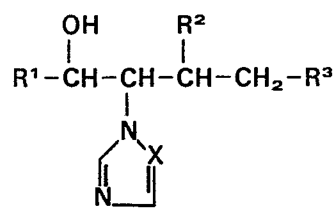

(Ib)

in welcher $R^1$, $R^2$, $R^3$ und X die oben angegebene Bedeutung haben, stellen interessante Zwischenprodukte dar. Sie können in üblicher Weise an der OH-Gruppe derivatisiert werden, wie beispielsweise verethert, acyliert oder carbamoyliert.

Die neuen Azolylalkylderivate der Formel I weisen starke fungizide Eigenschaften auf. Dabei zeigen überraschenderweise die erfindungsgemässen Verbindungen eine erheblich höhere Wirkung als bekannte Azolderivate, wie beispielsweise die eingangs genannten, welche wirkungsmässig naheliegende Verbindungen sind. Die erfindungsgemässen Stoffe stellen somit eine Bereicherung der Technik dar.

Die erfindungsgemässen Azolylalkylderivate sind durch die Formel I allgemein definiert.

Bevorzugt sind diejenigen Verbindungen der Formel I, in denen $R^1$ für tert.-Butyl, Isopropyl sowie für gegebenenfalls durch Fluor, Chlor, Methyl, Isopropyl, Methoxy, Methylthio, Trifluormethyl oder Phenyl substituiertes Phenyl steht; $R^2$ für Wasserstoff, Methyl, Ethyl, n-Propyl, Isopropyl sowie für gegebenenfalls durch Fluor, Chlor, Methyl, Ethyl, Isopropyl, Methoxy oder Phenyl substituiertes Phenyl steht; $R^3$ für Cyano oder die Gruppierung $-CO-OR^4$ steht; $R^4$ für Methyl, Ethyl, Propyl sowie für gegebenenfalls durch Fluor, Chlor oder Methyl substituiertes Benzyl steht; sowie A und X die in der Erfindungsdefinition angegebenen Bedeutungen haben.

Im einzelnen seien ausser den bei den Herstellungsbeispielen genannten Verbindungen die folgenden Verbindungen der allgemeinen Formel I genannt:

| $R^1$ | A | $R^2$ | $R^3$ | X |
|---|---|---|---|---|
| $(H_3C)_3C$ | CO | $CH_3$ | $-CO-OC_2H_5$ | N |
| $(H_3C)_3C$ | CH(OH) | $CH_3$ | $-CO-OCH_3$ | N |
| $(H_3C)_3C$ | CO | $CH_3$ | $-CO-OC_2H_5$ | CH |
| $(H_3C)_3C$ | CH(OH) | $CH_3$ | $-CO-OCH_3$ | CH |
| 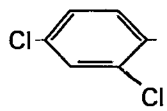 | CO | H | $-CN$ | N |
| 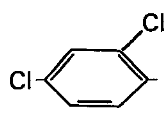 | CH(OH) | H | $-CN$ | N |
| 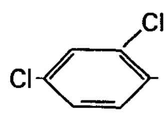 | CO | H | $-CN$ | CH |
| 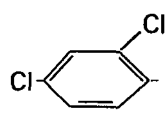 | CH(OH) | H | $-CN$ | CH |
| $(H_3C)_3C$ | CH(OH) | $C_2H_5$ | $-CO-OC_2H_5$ | N |
| $(H_3C)_3C$ | CO | $C_3H_7$-n | $-CO-OC_2H_5$ | N |
| $(H_3C)_3C$ | CO | $C_3H_7$-iso | $-CO-OC_2H_5$ | N |
| $(H_3C)_3C$ | CO | 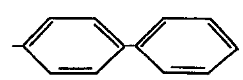 | $-CO-OCH_3$ | N |
| $(H_3C)_3C$ | CO | 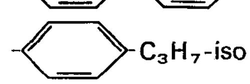 | $-CO-OCH_3$ | N |

Verwendet man beispielsweise Imidazol-1-ylpinakolin und Zimtsäurenitril als Ausgangsstoffe, so kann der Reaktionsablauf durch das folgende Formelschema wiedergegeben werden:

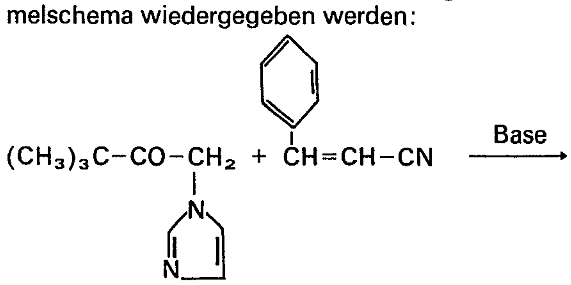


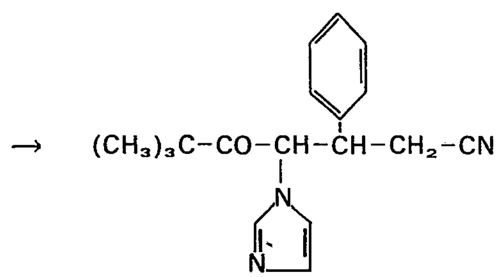

Verwendet man beispielsweise 6-Cyano-2,2-dimethyl-4-imidazol-1-yl-5-phenyl-3-hexanon und Natriumborhydrid als Ausgangsstoffe, so

kann der Rekationsablauf durch das folgende Formelschema wiedergegeben werden (Reduktionsverfahren):

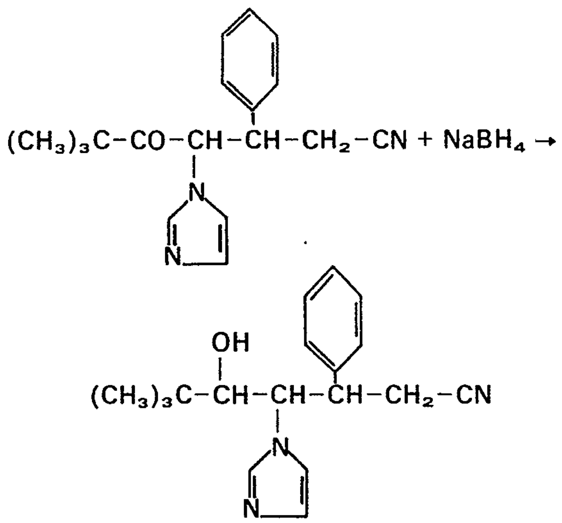

Die bei der Durchführung des erfindungsgemässen Verfahrens als Ausgangstoffe zu verwendenden Azolylketone sind durch die Formel II allgemein definiert. In dieser Formel stehen $R^1$ und X vorzugsweise für die Reste, die bei der Beschreibung der erfindungsgemässen Stoffe der Formel I bereits vorzugsweise für diese Substituenten genannt wurden.

Die Azolylketone der Formel II sind bekannt (vgl. DE-OS Nrn. 2431407, 2610022 und 2638470). Sie lassen sich nach üblichen Methoden herstellen, indem man die entsprechenden Halogenketone in Gegenwart eines Säurebinders mit 1,2,4-Triazol oder Imidazol umsetzt.

Die ausserdem für das erfindungsgemässe Verfahren als Ausgangsstoffe zu verwendenden Alkene sind durch die Formel III allgemein definiert. In dieser Formel stehen $R^2$ und $R^3$ vorzugsweise für die Reste, die bei der Beschreibung der erfindungsgemässen Stoffe der Formel I bereits vorzugsweise für diese Substituenten genannt wurden.

Die Alkene der Formel III sind allgemein bekannte Verbindungen der organischen Chemie.

Für die erfindungsgemässen Umsetzungen kommen als Verdünnungsmittel organische Lösungsmittel in Frage. Hierzu gehören vorzugsweise Alkohole, wie Methanol, Ethanol oder Isopropanol; aromatische Kohlenwasserstoffe, wie Benzol oder Toluol; Ether, wie Tetrahydrofuran oder Dioxan; sowie Nitrile, wie Acetonitril.

Die erfindungsgemässen Umsetzungen werden in Gegenwart einer Base durchgeführt. Hierzu gehören vorzugsweise Alkalialkoholate, wie Natriummethylat, Natriumethylat oder Kaliummethylat; Alkalimetallhydroxide, wie Natrium- oder Kaliumhydroxid; sowie Alkalimetallfluoride, wie Natrium- oder Kaliumfluorid.

Die erfindungsgemässen Umsetzungen werden gegebenenfalls in Gegenwart eines Katalysators durchgeführt. Hierzu gehören vorzugsweise alle üblichen macrocyclischen Polyether (Kronenverbindungen).Die Reaktionstemperaturen können bei der Durchführung der Verfahren in einem grösseren Bereich variiert werden. Im allgemeinen arbeitet man zwischen 10 und 150°C, vorzugsweise bei der Siedetemperatur des verwendeten Lösungsmittels.

Bei der Durchführung des erfindungsgemässen Verfahrens arbeitet man vorzugsweise in molaren Mengen. Die Isolierung der Verbindung der Formel I erfolgt in üblicher Art und Weise.

Die erfindungsgemässe Reduktion erfolgt in üblicher Weise, wie z.B. durch Umsetzung mit komplexen Hydriden, gegebenenfalls in Gegenwart eines Verdünnungsmittels.

Arbeitet man mit komplexen Hydriden, so kommen als Verdünnungsmittel für die erfindungsgemässe Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30°C, vorzugsweise bei 0 bis 20°C durchgeführt. Hierzu setzt man auf 1 mol des Ketons der Formel Ia etwa 1 mol eines komplexen Hydrids, wie Natriumborhydrid oder Lithiumalanat, ein. Zur Isolierung der reduzierten Verbindungen der Formel I wird der Rückstand in verdünnter Salzsäure aufgenommen, anschliessend alkalisch gestellt und mit einem organischen Lösungsmittel extrahiert. Die weitere Aufarbeitung erfolgt in üblicher Weise.

Zur Herstellung von physiologisch verträglichen Säureadditionssalzen der Verbindungen der Formel I kommen vorzugsweise folgende Säuren in Frage: die Halogenwasserstoffsäuren, wie z.B. die Chlor- und die Bromwasserstoffsäure, insbesondere die Chlorwasserstoffsäure, ferner Phosphor-, Salpeter-, Schwefelsäure, mono- und bifunktionelle Carbon- und Hydroxycarbonsäuren, wie z.B. Essig-, Malein-, Bernstein-, Fumar-, Wein-, Zitronen-, Salicyl-, Sorbin-, Milchsäure, sowie Sulfonsäuren, wie z.B. p-Toluolsulfonsäure und 1,5-Naphthalindisulfonsäuren. Die Säureadditionssalze der Verbindungen der Formel I können in einfacher Weise nach üblichen Salzbildungsmethoden, z.B. durch Lösen einer Verbindung der Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalzkomplexen der Verbindungen der Formel I kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. die Chlor- und die Bromwasserstoffsäure, ferner Phoshor-, Salpeter- und Schwefelsäure.

Die Metallsalzkomplexe von Verbindungen der Formel I können in einfacher Weise nach üblichen Verfahren erhalten werden, so z.B. durch Lösen des Metallsalzes in Alkohol, z.B. Ethanol, und Hinzufügen zur Verbindung der Formel I. Man kann Metallsalzkomplexe in bekannter Weise, z.B. durch Abfiltrieren, Isolieren und gegebenenfalls durch Umkristallisieren reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromyzetes, Oomyzetes, Chytridiomyzetes, Zygomyzetes, Ascomyzetes, Basidiomyzetes, Deuteromyzetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung von Getreidekrankheiten, wie Rost und Mehltau; von *Venturia*-Arten, wie gegen den Erreger des Apfelschorfs *(Fusicladim dendriticum)*; sowie von *Podosphaera*-Arten, wie gegen den Erreger des echten Apfelmehltaus *(Podosphaera leucotricha)* eingesetzt werden.

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylenfettsäureester, Polyoxyethylenfettalkoholether, z.B. Alkylarylpolyglykolether, Alkylsulfonate, Alkylsulfate, Arylsulfonat sowie Eiweisshydrolysate; als Dispergiermittelkommen in Frage: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink erwendet werden.

Die Formulierungen enthalten im allgemeinen zwischen 0,1 und 95 Gew.-% Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trokken-, Feucht-, Nass-, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g/kg Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

*Herstellungsbeispiele*

*Beispiel 1:*

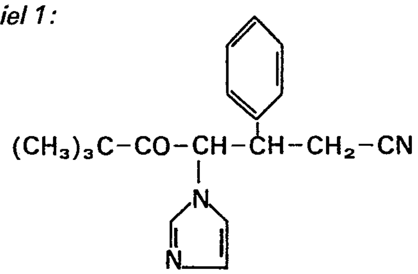

25,8 g (0,2 mol) Zimtsäurenitril und 33,2 g (0,2 mol) Imidazol-1-ylpinakolin in 200 ml Methanol werden mit 6,6 g Natriummethylat in 66 ml Methanol versetzt und 44 h unter Rückfluss erhitzt. Danach wird das Lösungsmittel im Vakuum abdestilliert, der Rückstand in Wasser aufgenommen und mit 2N Salzsäure neutral gestellt. Die wässerige Phase wird zweimal mit je 100 ml Methylenchlorid extrahiert, die organische Phase anschliessend dreimal mit je 100 ml Wasser gewaschen, über Natriumsulfat getrocknet, eingeengt und der Rückstand in 500 ml Aceton gelöst. Man gibt 36 g 1,5-Naphthalindisulfonsäure, gelöst in 200 ml Aceton, zu und saugt den entstanderen Niederschlag ab. Dieser wird in 500 ml Methylenchlorid aufgenommen und mit 1 l gesättigter Natriumhydrogencarbonatlösung verrührt. Die organische Phase wird abgetrennt, mit 1 l Wasser gewaschen, über Natrimsulfat getrocknet und eingeengt. Der ölige Rückstand wird in Ether aufgenommen, wobei es zur Kristallisation kommt. Man erhält 34 g (57,6% der Theorie) 6-Cyano-2,2-dimethyl-4-imidazol-1-yl-5-phenyl-6-hexanon als Diastereomerengemisch von Smp. 108-111°C.

*Beispiel 2:*

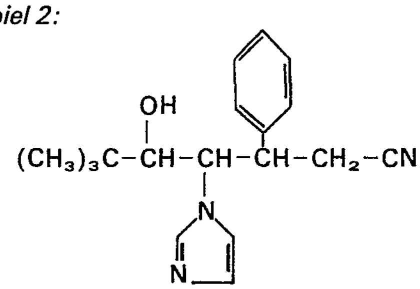

96,5 g (0,33 mol) 6-Cyano-2,2-dimethyl-4-imidazol-1-yl-5-phenyl-6-hexanon (Beispiel 1) werden in 400 ml Isopropanol gelöst und bei 1 bis 20°C portionsweise mit 15,12 g (0,4 mol) Natriumborhydrid versetzt. Man lässt 24 h bei Raumtemperatur nachrühren, versetzt bei 0 bis 10°C tropfenweise mit 800 ml 2N Salzsäure und lässt erneut 24 h bei Raumtemperatur nachrühren. Anschliessend wird das Reaktionsgemisch mit 1,5 l gesättigter Natriumhydrogencarbonatlösung neutralisiert und dreimal mit je 400 ml Methylenchlorid extrahiert. Die vereinigten organischen Phasen werden mit Wasser gewaschen, über Natriumsulfat getrocknet und eingeengt. Der Rückstand wird in 300 ml Diisopropylether aufgekocht und die beim Abkühlen entstehenden Kristalle abgesaugt. Man erhält 28,7 g (30% der Theorie) 6-Cyano-2,2-dimethyl-4-imidazol-1-yl-5-phenyl-6-hexanol als Diastereomerengemisch vom Smp. 212-228°C.

In entsprechender Weise werden die nachfolgenden Beispiele der allgemeine Formel:

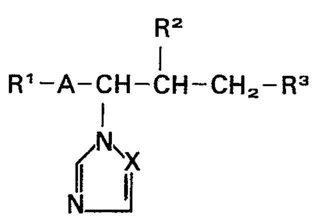

(I)

erhalten:

| Beispiel Nr. | $R^1$ | A | X | $R^2$ | $R^3$ | Fp. (°C) |
|---|---|---|---|---|---|---|
| 3 | $(CH_3)_3C$ | CO | CH | H | CN | Kp: 170°C/0,05 mm |
| 4 | $(CH_3)_3C$ | CO | CH | 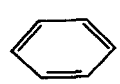 | $-CO-OCH_3$ | 118-132 |
| 5 | $(CH_3)_3C$ | CO | CH | 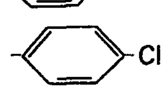 | $-CO-OCH_3$ | 160-166 |
| 6 | $(CH_3)_3C$ | CO | CH | 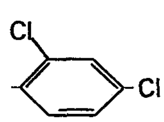 | $-CO-OC_2H_5$ | 88(Z) (×HCl) |

| Beispiel Nr. | $R^1$ | A | X | $R^2$ | $R^3$ | Fp. (°C) |
|---|---|---|---|---|---|---|
| 7 | $(CH_3)_3C$ | CO | CH | 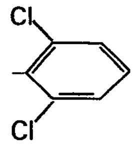 | $-CO-OCH_3$ | 180-202(Z) (×HCl) |
| 8 | $(CH_3)_3C$ | CO | CH | 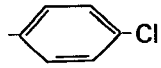 | CN | 218-243 |
| 9 | $(CH_3)_3C$ | CO | CH | 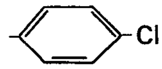 | $-CO-OC_2H_5$ | 57-70(Z) (×HCl) |
| 10 | $(CH_3)_3C$ | CH(OH) | CH | H | CN | 148-152 |
| 11 | $(CH_3)_3C$ | CH(OH) | CH | 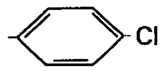 | CN | >280 (×½ NDS) |
| 12 | $(CH_3)_3C$ | CH(OH) | CH | 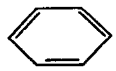 | $-CO-OCH_3$ | 191-198(Z) |
| 13 | $(CH_3)_3C$ | CH(OH) | CH | 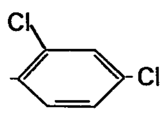 | $-CO-OC_2H_5$ | 130(Z) (×HCl) |
| 14 | $(CH_3)_3C$ | CH(OH) | CH | 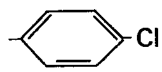 | $-CO-OC_2H_5$ | 270(Z) |
| 15 | $(CH_3)_3C$ | CO | N | H | CN | 62-66 |
| 16 | $(CH_3)_3C$ | CO | N | 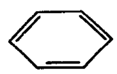 | CN | 120-124 |
| 17 | $(CH_3)_3C$ | CO | N | 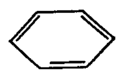 | $-CO-OCH_3$ | 93-106 |
| 18 | $(CH_3)_3C$ | CO | N | 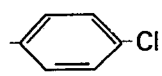 | $-CO-OCH_3$ | 112-114 |
| 19 | $(CH_3)_3C$ | CO | N | 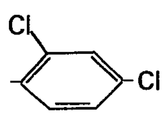 | $-CO-OC_2H_5$ | 128-138 |
| 20 | $(CH_3)_3C$ | CO | N | 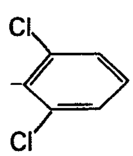 | $-CO-OCH_3$ | 108-132 |
| 21 | $(CH_3)_3C$ | CO | N | 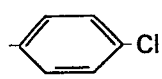 | CN | 132-152 |
| 22 | $(CH_3)_3C$ | CH(OH) | N | H | CN | 107-112 |
| 23 | $(CH_3)_3C$ | CH(OH) | N | 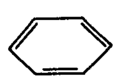 | CN | 123-126 |
| 24 | $(CH_3)_3C$ | CH(OH) | N | 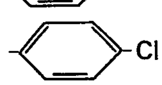 | CN | 78(Z) |
| 25 | $(CH_3)_3C$ | CH(OH) | N | 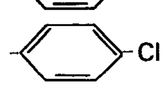 | $-CO-OCH_3$ | 68(Z) |
| 26 | $(CH_3)_3C$ | CH(OH) | N | 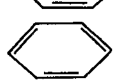 | $-CO-OCH_3$ | 52-58 |

*Verwendungsbeispiele:*

In den nachfolgenden Beispielen werden die nachstehend angegebenen Verbindungen als Vergleichssubstanzen eingesetzt:

(A) 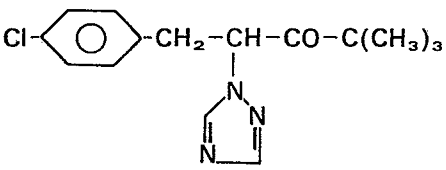

(B) 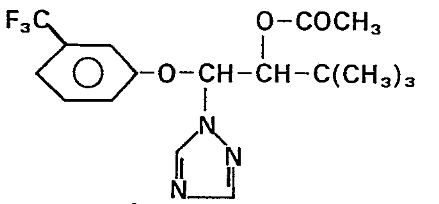

(C) 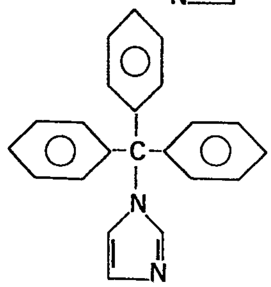

(D) 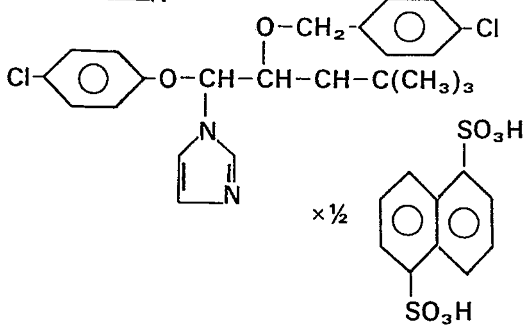

*Beispiel A:*

Podosphaera-*Test (Apfel)/protektiv*

Lösungsmittel: 4,7 Gew.-Teile Aceton

Emulgator: 0,3 Gew.-Teile Alkylarylpolyglykolether

Wasser: 95,0 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 h bei 20°C und einer relativen Luftfeuchtigkeit von 70% im Gewächshaus. Anschliessend werden sie durch Bestäuben mit Konidien des Apfelmehltauerregers *(Podosphaera leucotricha)* inokuliert und in ein Gewächshaus mit einer Temperatur von 21 bis 23°C und einer relativen Luftfeuchtigkeit von 70% gebracht.

10 d nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

Eine deutliche Überlegenheit in der Wirksamkeit gegenüber der aus dem Stand der Technik bekannten Verbindung A zeigen bei diesem Test z.B. die Verbindungen gemäss folgender Herstellungsbeispiele: 19, 20, 22 und 25.

*Beispiel B:*

Fusicladium-*Test (Apfel)/protektiv*

Lösungsmittel: 4,7 Gew.-Teile Aceton

Emulgator: 0,3 Gew.-Teile Alkylarylpolyglykolether

Wasser: 95,0 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 h bei 20°C und einer relativen Luftfeuchtigkeit von 70% im Gewächshaus. Anschliessend werden sie mit einer wässerigen Konidiensuspension des Apfelschorferregers *(Fusicladium dendriticum)* inokuliert und 18 h lang in einer Feuchtkammer bei 18 bis 20°C und 100% relativer Luftfeuchtigkeit inkubiert.

Die Pflanzen kommen dann erneut für 14 d ins Gewächshaus.

15 d nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

Eine deutliche Überlegenheit in der Wirksamkeit gegenüber dem Stand der Technik bekannten Verbindungen A und C zeigen bei diesem Test z.B. die Verbindungen gemäss folgender Herstellungsbeispiele: 19, 4, 1, 5, 8, 9, 11 und 2.

*Beispiel C:*

Erysiphe-*Test (Gerste)/protektiv*

Lösungsmittel: 100 Gew.-Teile Dimethylformamid

Emulgator: 0,25 Gew.-Teile Alkylarylpolyglykolether

Zur Herstellung einer zweckmässigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von *Erysiphe graminis* f. sp. *hordei* bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 d nach der Inokulation erfolgt die Auswertung. Eine deutliche überlegenheit in der Wirksamkeit gegenüber dem Stand der Technik zeigen bei diesem Test z.B. die Verbindungen gemäss folgender Herstellungsbeispiele: 4, 5, 6, 7, 8, 9, 12, 13, 14, 17, 18, 19, 20, 22, 23, 24 und 25.

## Patentansprüche

1. Azolylalkylderivate der allgemeinen Formel:

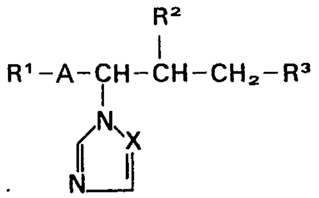 (I)

in welcher

$R^1$ für Alkyl mit 1 bis 4 Kohlenstoffatomen oder für Phenyl steht, wobei letzteres durch Halogen, Alkyl mit 1 bis 4 Kohlenstoffatome, Halogenalkyl mit 1 oder 2 Kohlenstoff- und 1 bis 5 Halogenatome, durch Alkoxy und Alkylthio mit jeweils 1 oder 2 Kohlenstoffatomen sowie durch Phenyl substituiert sein kann,

$R^2$ für Wasserstoff, für Alkyl mit 1 bis 4 Kohlenstoffatomen oder für Phenyl steht, wobei letzteres durch die für $R^1$ genannten Phenylsubstituenten substituiert sein kann,

$R^3$ für Cyano oder die Gruppierung $-CO-OR^4$ steht, wobei $R^4$ für Alkyl mit 1 bis 4 Kohlenstoffatomen oder für Benzyl steht, wobei letzteres durch die für $R^1$ genannten Phenylsubstituenten substituiert sein kann,

A für die Ketogruppe oder die CH(OH)-Gruppierung steht, und

X für ein Stickstoffatom oder die CH-Gruppe steht,

sowie deren physiologisch verträgliche Säureadditionssalze und Metallsalzkomplexe.

2. Verbindungen der Formel (I) in Anspruch 1, wobei

$R^1$ für tert.-Butyl, Isopropyl sowie für gegebenenfalls durch Fluor, Chlor, Methyl, Isopropyl, Methoxy, Methylthio, Trifluormethyl oder Phenyl substituiertes Phenyl steht,

$R^2$ für Wasserstoff, Methyl, Ethyl, n-Propyl, Isopropyl sowie für gegebenenfalls durch Fluor, Chlor, Methyl, Ethyl, Isopropyl, Methoxy oder Phenyl substituiertes Phenyl steht,

$R^3$ für Cyano oder die Gruppierung $-CO-OR^4$ steht, wobei $R^4$ für Methyl, Ethyl, Propyl sowie für gegebenenfalls durch Fluor, Chlor oder Methyl substituiertes Benzyl steht, und A und X die in Anspruch 1 angegebenen Bedeutungen haben.

3. Verfahren zur Herstellung der Azolylalkylderivate der Formel:

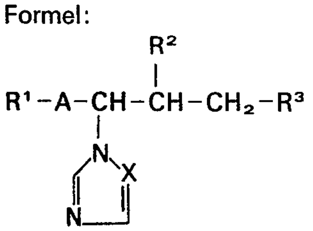 (I)

in welcher

$R^1$ für Alkyl mit 1 bis 4 Kohlenstoffatomen oder für Phenyl steht, wobei letzteres durch Halogen, Alkyl mit 1 bis 4 Kohlenstoffatomen, Halogenalkyl mit 1 oder 2 Kohlenstoff- und 1 bis 5 Halogenatomen, durch Alkoxy und Alkylthio mit jeweils 1 oder 2 Kohlenstoffatomen sowie durch Phenyl substituiert sein kann,

$R^2$ für Wasserstoff, für Alkyl mit 1 bis 4 Kohlenstoffatomen oder für Phenyl steht, wobei letzteres durch die für $R^1$ genannten Phenylsubstituenten substituiert sein kann,

$R^3$ für Cyano oder die Gruppierung $-CO-OR^4$ steht, wobei $R^4$ für Alkyl mit 1 bis 4 Kohlenstoffatomen oder für Benzyl steht, wobei letzteres durch die für $R^1$ genannten Phenylsubstituenten substituiert sein kann,

A für die Ketogruppe oder die CH(OH)-Gruppierung steht, und

X für ein Stickstoffatom oder die CH-Gruppe steht,

dadurch gekennzeichnet, dass man Azolylketone der Formel:

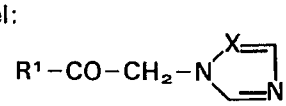 (II)

in welcher $R^1$ und X die oben angegebene Bedeutung haben,

mit Alkenen der Formel:

$$R^2-CH=CH-R^3 \qquad (III)$$

in welcher $R^2$ und $R^3$ die oben angegebene Bedeutung haben,

in Gegenwart eines Verdünnungsmittels und in Gegenwart einer Base sowie gegebenenfalls in Gegenwart eines Katalysators umsetzt und gegebenenfalls die entstehenden Azolylalkylketoderivate der Formel:

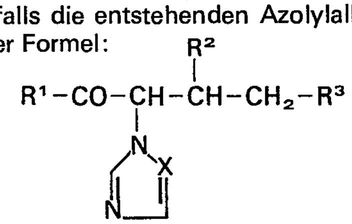 (Ia)

in welcher $R^1$, $R^2$, $R^3$ und X die oben angegebene Bedeutung haben,

nach bekannten Methoden in üblicher Weise reduziert.

4. Fungizide Mittel, gekennzeichnet durch einen Gehalt an mindestens einem Azolylalkylderivat der Formel (I) in den Ansprüchen 1 und 3.

5. Verwendung von Azolylalkylderivaten der Formel (I) in den Ansprüchen 1 und 3 zur Bekämpfung von pflanzenpathogenen Pilzen.

6. Verfahren zur Herstellung fungizider Mittel, dadurch gekennzeichnet, dass man Azolylalkylderivate der Formel (I) in den Ansprüchen 1 und 3 mit Streckmitteln und/oder oberflächenaktiven Mitteln vermischt.

## Claims

1. Azolylalkyl derivatives of the general formula:

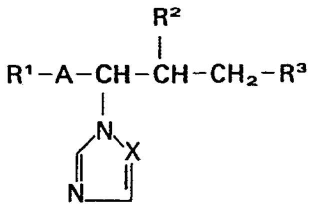

(I)

in which

$R^1$ represents alkyl with 1 to 4 carbon atoms or phenyl, it being possible for the latter to be substituted by halogen, alkyl with 1 to 4 carbon atoms, halogenoalkyl with 1 on 2 carbon and 1 to 5 halogen atoms, by alkoxy or alkylthio with in each case 1 or 2 carbon atoms, or by phenyl,

$R^2$ represents hydrogen, alkyl with 1 to 4 carbon atoms or phenyl, it being possible for the latter to be substituted by the substituents on phenyl mentioned in the case of $R^1$,

$R^3$ represents cyano or the grouping $-CO-OR^4$, wherein $R^4$ represents alkyl with 1 to 4 carbon atoms or benzyl, it being possible for the latter to be substituted by the substituents on phenyl mentioned in the case of $R^1$,

A represents the keto group or the CH(OH) grouping, and

X represents a nitrogen atom or the CH group, and physiologically acceptable acid addition salts and metal salt complexes thereof.

2. Compounds of the formula (I) in Claim 1, wherein

$R^1$ reprsents tert.-butyl, isopropyl, or phenyl which is optionally substituted by fluorine, chlorine, methyl, isopropyl, methoxy, methylthio, trifluoromethyl, or phenyl,

$R^2$ represents hydrogen, methyl, ethyl, n-propyl, isopropyl, or phenyl which is optionally substituted by fluorine, chlorine, methyl, ethyl, isopropyl, methoxy, or phenyl,

$R^3$ represents cyano or the grouping $-CO-OR^4$, wherein $R^4$ represents methyl, ethyl, propyl, or benzyl which is optionally substituted by fluorine, chlorine, or methyl, and

A and X have the meanings indicated in Claim 1.

3. Process for the preparation of the azolylalkyl derivatives of the formula:

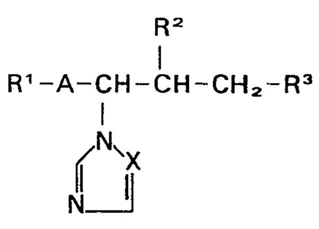

(I)

in which

$R^1$ represents alkyl with 1 to 4 carbon atoms or phenyl, it being possible for the latter to be substituted by halogen, alkyl with 1 to 4 carbon atoms, halogenoalkyl with 1 or 2 carbon and 1 to 5 halogen atoms, by alkoxy or alkylthio with in each case 1 or 2 carbon atoms, or by phenyl,

$R^2$ represents hydrogen, alkyl with 1 to 4 carbon atoms or phenyl, it being possible for the latter to be substituted by the substituents on phenyl mentioned in the case of $R^1$,

$R^3$ represents cyano or the grouping $-CO-OR^4$, wherein $R^4$ represents alkyl with 1 to 4 carbon atoms or benzyl, it being possible for the latter to be substituted by the substituents on phenyl mentioned in the case of $R^1$,

A represents the keto group or the CH(OH) grouping, and

X represents a nitrogen atom or the CH group, characterised in that azolyl-ketones of the formula:

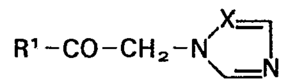

(II)

in which $R^1$ and X have the meaning indicated above,

are reacted with alkenes of the formula:

$$R^2-CH=CH-R^3 \quad (III)$$

in which $R^2$ and $R^3$ have the meaning indicated above,

in the presence of a diluent and in the presence of a base and if approriate in the presence of a catalyst and, optionally, the azolylalkyl-keto derivatives formed, of the formula:

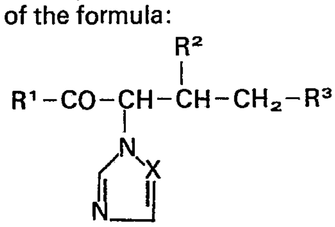

(Ia)

in which $R^1$, $R^2$, $R^3$ and X have the meaning indicated above,

are reduced in a conventional manner by known methods.

4. Fungicidal agents, characterised in that they contain at least one azolylalkyl derivative of the formula (I) in Claims 1 and 3.

5. Use of azolylalkyl derivatives of the formula (I) in Claims 1 and 3 for combating phytopathogenic fungi.

6. Process for the preparation of fungicidal agents, characterised in that azolylalkyl derivatives of the formula (I) in Claims 1 and 3 are mixed with extenders and/or surface-active agents.

**Revendications**

1. Dérivés d'azolylalkyles de formule générale:

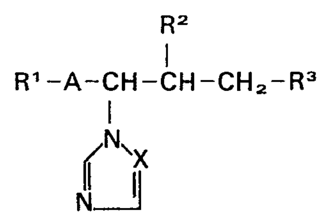

(I)

dans laquelle:

$R^1$ représente un groupe alkyle ayant 1 à 4 atomes de carbone ou un groupe phényle, ce dernier pouvant être substitué par de l'halogène, par un groupe alkyle ayant 1 à 4 atomes de carbone, halogénoalkyle ayant 1 ou 2 atomes de carbone et 1 à 5 atomes d'halogène, par un groupe

alcoxy et alkylthio ayant chacun 1 ou 2 atomes de carbone, ainsi que par un groupe phényle,

$R^2$ représente de l'hydrogène, un groupe alkyle ayant 1 à 4 atomes de carbone ou un groupe phényle, ce dernier pouvant être substitué par les substituants cités pour $R^1$ à propos des substituants du groupe phényle,

$R^3$ représente un groupe cyano ou le groupement $-CO-OR^4$, où $R^4$ représente un groupe alkyle ayant 1 à 4 atomes de carbone ou un groupe benzyle, ce dernier pouvant être substitué par les substituants du groupe phényle cités pour $R^1$,

A représente un groupe céto ou le groupe CH(OH), et

X représente un atome d'azote ou le grouep CH, ainsi que leurs sels d'addition d'acide et complexes de sels de métaux physiologiquement acceptables.

2. Composés de formule (I) de la revendication 1, dans laquelle:

$R^1$ représente un groupe tertiobutyle, isopropyle, ainsi que phényle (éventuellement substitué par du fluor, du chlore, un groupe méthyle, isopropyle, méthoxy, méthylthio, trifluorométhyle ou phényle),

$R^2$ représente de l'hydrogène, un groupe méthyle, éthyle, n-propyle, isopropyle, ainsi qu'un groupe phényle (éventuellement substitué par du fluor, du chlore, un groupe méthyle, éthyle, iospropyle, méthoxy ou phényle),

$R^3$ représente un groupe cyano ou le groupement $-CO-OR^4$, où $R^4$ représente un groupe méthyle, éthyle, propyle, ainsi qu'un groupe benzyle (éventuellement substitué par du fluor, du chlore ou un groupe méthyle), et

A et X ont les sens indiqués à la revendication 1.

3. Procédé de préparation des dériés d'azolylalkyles de formule (I):

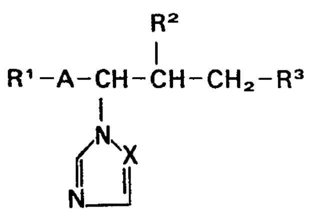

(I)

dans laquelle:

$R^1$ représente un groupe alkyle ayant 1 à 4 atomes de carbone ou un groupe phényle, ce dernier pouvant être substitué par de l'halogène, un groupe alkyle ayant 1 à 4 atomes de carbone, halogénoalkyle ayant 1 ou 2 atomes de carbone et 1 à 5 atomes d'halogène, par un groupe alcoxy et alkylthio ayant chacun 1 ou 2 atomes de carbone, ainsi que par un groupe phényle,

$R^2$ représente de l'hydrogène, un groupe alkyle ayant 1 à 4 atomes de carbone ou un groupe phényle, ce dernier pouvant être substitué par les substituants cités pour le groupe phényle à propos de $R^1$,

$R^3$ représente un groupe cyano ou le groupement $-CO-OR^4$, où $R^4$ représente un groupe alkyle ayant 1 à 4 atomes de carbone ou un groupe benzyle, ce dernier pouvant être substitué par les substituants du groupe phényle cités pour $R^1$,

A représente le groupe céto ou le groupement CH(OH), et

X représente un atome d'azote ou le groupe CH, procédé caractérisé en ce qu'on fait réagir, en présence d'un diluant et en présence d'une base et éventuellement aussi en présence d'un catalyseur, des azolylcétones de formule:

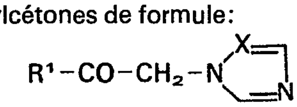

(II)

dans laquelle $R^1$ et X ont le sens indiqué ci-dessus, avec des alcènes de formule:

$$R^2-CH=CH-R^3 \quad (III)$$

dans laquelle $R^2$ et $R^3$ ont le sens indiqué ci-dessus,

et éventuellement on réduit de façon connue, selon les méthodes connues, les dérivés azolylalkylcétoniques résultants, de formule:

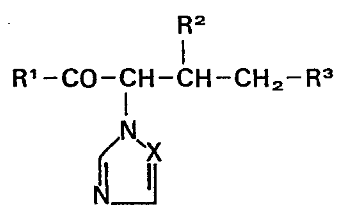

(Ia)

dans laquelle $R^1$, $R^2$, $R^3$ et X ont le sens indiqué ci-dessus.

4. Fongicide, caractérisé en ce qu'il contient au moins un dérivé d'azolylalkyle de formule (I) selon les revendications 1 et 3.

5. Application de dérivés d'azolylalkyles de formule (I) selon les revendications 1 et 3 à la lutte contre les champignons phytopathogènes.

6. Procédé de préparation de fongicides, caractérisé en ce qu'on mélange des dérivés d'azolylalkyles de formule (I) selon les revendications 1 et 3 avec des agents d'allongement et/ou avec des tensio-actifs.